# EUROPEAN PATENT APPLICATION

(11) **EP 1 997 892 A1**
(43) Date of publication of application: **03.12.2008**
(21) Application number: 08156793.5
(22) Date of filing: 23.05.2008
(51) Int. Cl.: C12N 15/10

(54) **Screening for expressible transfectants in a eukaryotic system**

(30) Priority: 25.05.2007 DK 200700765
(71) Applicant: Symphogen A/S, 2800 Lyngby (DK)
(72) Inventor: Nielsen, Lars Søgård, 2990, Nivå (DK); Meijer, Per-Johan, 2800, Kgs. Lyngby (DK)

(57) **Abstract**

The present invention relates to the field of molecular cloning and to the field of expression cloning in higher, eukaryotic cells. In particular, the present invention relates to a method for fast and reliable identification of vectors and vector DNA which will be capable of providing a desired expression product if a eukaryotic host cell is transfected with the vector DNA. The invention allows automated DNA amplification of cDNA libraries arrayed in multi-well plates. The method significantly decreases the complexity and time consumption for performing High throughput expression cloning of secreted proteins. In principle, the use of amplified DNA for transfections will simplify and thereby increase the throughput of procedures that include transfection of mammalian cells in multi-well format with a catalogued or arrayed library.

## Description

### FIELD OF THE INVENTION

The present invention relates to the field of molecular cloning and to the field of expression cloning in higher, eukaryotic cells. In particular, the present invention relates to a method for fast and reliable identification of vectors and vector DNA which will be capable of providing a desired expression product if a eukaryotic host cell is transfected with the vector DNA.

### BACGROUND OF THE INVENTION

### Functional screening

Expression cloning and functional genomics approaches aim to identify proteins that confer or contribute to a selectable or detectable activity. This has been addressed by several different methods ranging from sophisticated gene-trap analysis in organisms (reviewed in [Durick K, Mendlein J and Xanthopoulos KG. Hunting with traps: genome-wide strategies for gene discovery and functional analysis. Genome Res, 1999;9:1019-1025]) through high throughput interaction studies [Pearl berg J and LaBaer J. Protein expression clone repositories for functional proteomics. Curr Opin Chem Biol, 2004;8:98-102].

One successful approach for identification of proteins and assignment of gene function has been mammalian cell based expression cloning. In this method, cDNA derived from different cell lines or tissues are cloned as a library into a viral or plasmid based expression vector and transfected transiently or permanently into an appropriate mammalian cell line. A selection or screening for cDNA encoding genes exerting either an intracellular function of interest, e.g. resistance to induction of apoptosis, induction of proliferation, panning of cells expressing a cell-surface receptor or inducing an activity can be performed [Seed B and Aruffo A. Molecular cloning of the CD2 antigen, the T-cell erythrocyte receptor, by a rapid immunoselection procedure. Proc Natl Acad Sci U S A, 1987;84:3365-3369; Stark GR and Gudkov AV. Forward genetics in mammalian cells: functional approaches to gene discovery. Hum Mol Genet, 1999;8:1925-1938]. After isolation of cell lines with desired characteristics the DNA is recovered either by plasmid isolation techniques if transient transfection is used or by for example PCR techniques if transfected DNA has been inserted into the genome and the gene of interest is identified. This approach allows single-vessel transfections since the DNA is physically combined with the phenotype of interest. In addition the cDNA libraries are expressed in mammalian cells, i.e. as close to the natural environment as possible for human genes or proteins. These advantages also apply to mammalian display systems where a secreted protein (e.g. an antibody) is linked to a surface protein, e.g. a receptor fragment to ensure that the protein is linked to the surface of the cell expressing it. Mammalian cells displaying antibodies can be screened for binding to an antigen. Positive hits can be isolated using for example FACS and the cDNA can be obtained from the identified cell as described above.

The chemically or physically mediated DNA transfection methods employed for mammalian cells typically result in uptake of several plasmids per cell, which generates background during the screening and consequently a decreased sensitivity. Furthermore, situations where the effector protein consists of two or more subunits (e.g. antibody heavy and light chains) the existence of multiple plasmids encoding different library members will introduce scrambling between such protein subunits and thereby prevent efficient selection. Utilization of retroviral gene transfer to establish a single gene - single cell situation can to some extent eliminate these issues [Whitehead I, Kirk H and Kay R. Expression cloning of oncogenes by retroviral transfer of cDNA libraries. Mol Cell Biol, 1995; 15:704-710; Kitamura T. New experimental approaches in retrovirus-mediated expression screening. Int J Hematol, 1998;67:351-359]. An alternative method could be to employ site-specific integration methods with only a single integration site per cell.

Alternatively, if the aim of the screen is to identify secreted proteins such as cytokines or antibodies, there is no physical link between the cDNA and the phenotype an additional assay for identification of proteins with the desired reactivity will be required. Such expression cloning experiments requires catalogued or arrayed cDNA libraries in multi-well plates and individual transfections with each unique library clone; however this dramatically increases the workload and requires automated or semi-automated liquid handling systems for DNA preparation [Michiels F, van EH, van RL, Merchiers P, Francken B, Pittois K, van der SJ, Brys R, Vandersmissen J, Beirinckx F, Herman S, Dokic K, Klaassen H, Narinx E, Hagers A, Laenen W, Piest I, Pavliska H, Rombout Y, Langemeijer E, Ma L, Schipper C, Raeymaeker MD, Schweicher S, Jans M, van BK, Tsang IR, van de SO, Tomme P, Arts GJ and Donker J. Arrayed adenoviral expression libraries for functional screening. Nature Biotechnol, 2002;20:1154-1157; Meijer PJ, Andersen PS, Haahr HM, Steinaa L, Jensen A, Lantto J, Oleksiewicz MB, Tengbjerg K, Poulsen TR, Coljee VW, Bregenholt S, Haurum JS and Nielsen LS. Isolation of human antibody repertoires with preservation of the natural heavy and light chain pairing. J Mol Biol, 2006;358:764-772], cf. also WO 2005/042774, which i.a. relates to the identification of antibodies. In the past this has been achieved by dividing the library into sub-pools combined with sequential screening rounds leading to isolation and identification of the active gene. This approach has been successfully used for several cloning projects among these identification of cell growth factors [Lee F, Yokota T, Otsuka T, Gemmell L, Larson N, Luh J, Arai K and Rennick D. Isolation of cDNA for a human granulocyte-macrophage colony-stimulating factor by functional expression in mammalian cells. Proc Natl Acad Sci U S A, 1985;82:4360-4364; Yokota T, Lee F, Rennick D, Hall C, Arai N, Mosmann T, Nabel G, Cantor H and Arai K. Isolation and characterization of a mouse cDNA clone that expresses mast-cell growth-factor activity in monkey cells. Proc Natl Acad Sci U S A, 1984;81:1070-1074]. However, breaking the library into sub-pools will dilute the desired gene products which in some instances will be detrimental to the procedures.

### Current problems with expression cloning

Implementation of modern technology in biological assays has facilitated high throughput performance of biological reactions for screening of compound libraries. The more robust and simple the reaction, the more applicable is the assay to automation. In respect to screening of cDNA libraries the expression step requires DNA preparation for the transfection of the host cell. The DNA preparation is a key step that directly impacts the success rate. Usually plasmid DNA is used for transfection both prokaryote and eukaryote cells. Modern standards for plasmid extraction from *E. coli* utilises DNA binding resins for plasmid purification from the bacterial lysate. The overall procedure can be automated and performed in 96-well plates but the procedure consists of multiple steps such as centrifugation and/or vacuum aspiration and/or magnetic bead separation. From an automation perspective this is a complicated process that limits the throughput and results in a less robust procedure.

### Use of rolling circle amplification in screening

WO 2004/013350 (Novozymes) concerns methods for rapid and efficient industrial exploitation of relevant secreted or surface-displayed polypeptides from new organisms. The methods involve rolling circle amplification and signal tapping of libraries, wherein rolling circle amplification is used to amplify a pool of cDNAs isolated e.g. from an microbiological species. The amplified DNA, that consists of concatemers, can be cut with a restriction enzyme to isolate monomers, which after purification can be inserted into a bacterial cell for screening. The examples show that a cDNA library from Rhizomucor pusillus can be amplified using Phi29 (TempliPhi^{™}), cut with a restriction enzyme and subsequently be inserted into E. coli for further screening.

WO 2005/003389 (Royal Holloway and Bedford New College) concerns the problem that some DNA sequences appear to be "unclonable" using standard PCR based methods. The reference suggests that such unclonable DNA can be amplified using rolling circle amplification to produce a tandem series of repeats of the DNA fragment. Prior to transfection into host cells the amplified DNA may be cut with a restriction enzyme to isolate monomers of the amplified fragment.

The DNA polymerase used in the cited references is Phi29. Phi29 is available under the tradename TempliPhi® from Amersham and is described in US 5,198,543 (Consejo Superior Investigaciones Cientificas).

### OBJECT OF THE INVENTION

It is an object of the present invention to provide an improved method for identifying expression vectors which are capable of effecting expression of functional expression products.

### SUMMARY OF THE INVENTION

The present invention resides in the surprising finding that it is possible to utilise concatemers consisting of amplified plasmid DNA, obtained with the commercially available Templiphi® (PHI29 DNA polymerase, US 5,198,543) DNA template preparation method in an expression cloning like setup in eukaryotic cells, where the cells are transfected directly with the concatemers and subsequently tested for the presence of a desired expression product. This has in turn enabled an effective screening of expression product produced by multiple transfectants of eukaryotic cells without the drawbacks of first having to purify plasmid DNA by conventional labour intensive methods such as silica membrane or magnetic bead based methods.

In the present disclosure, we describe a simple single-step method utilizing polymerase-mediated DNA amplification for generation of DNA for transfection of mammalian cells. The invention allows automated DNA amplification of cDNA libraries arrayed in multi-well plates. The method significantly decreases the complexity and time consumption for performing High throughput expression cloning of secreted proteins. In principle, the use of amplified DNA for transfections will simplify and thereby increase the throughput of procedures that include transfection of mammalian cells in multi-well format with a catalogued or arrayed library.

Hence, in one aspect the present invention relates to a method for identifying and/or isolating vector nucleic acids, which each encodes an expression product with at least one predetermined functionality (EPPF) and which includes genetic elements sufficient to express said EPPF in a eukaryotic cell, the method comprising
a) providing a plurality of samples of vector nucleic acids, wherein each of said samples comprises one single vector nucleic acid species,
b) amplifying nucleic acids from each distinct sample in a molecular amplification procedure to produce amplification products being concatemers of said vector nucleic acid species,
c) transfecting each of said amplification products into a separate population of eukaryotic cells,
d) culturing each separate population of eukaryotic cells under conditions which facilitate expression of the amplification products,
e) subsequently testing for the presence of said EPPF in the cultures of eukaryotic cells, and
f) identifying and/or isolating the vector(s) of step (a) from which has/have been derived an EPPF found to be present in step e.

In a broader aspect, the invention relates to a method for identifying and/or isolating vector nucleic acids, which each encodes an expression product with at least one predetermined functionality (EPPF) and which includes genetic elements sufficient to express said EPPF in a eukaryotic cell, the method comprising
A) providing a plurality of nucleic acids each including a nucleotide sequence, which putatively encodes an EPPF,
B) amplifying, from each member of the plurality of nucleic acids, at least the nucleotide sequence which putatively encodes the EPPF in a molecular amplification procedure to produce amplification products being concatemers each including said nucleotide sequence in operable linkage with genetic elements sufficient to express said nucleotide sequence in a eukaryotic cell and,
C) transfecting each of said amplification products into eukaryotic cells,
D) culturing the eukaryotic cells under conditions which facilitate expression of the amplification products,
E) subsequently testing for the presence of said EPPF in the cultures of the eukaryotic cells, and
F) identifying and/or isolating a vector including the nucleotide sequence encoding an EPPF found to be present in step E).

In a third aspect, the present invention relates to a method for producing an EPPF in eukaryotic cells, the method comprising identifying a vector nucleic acid by means of the identification methods of the invention, and subsequently transfecting or transducing a suitable eukaryotic host cell with the vector from where the vector nucleic acid is derived, culturing the transfected or transduced eukaryotic cell under conditions which facilitate expression of the EPPF encoding nucleic acid and recovering the EPPF from the culture

### LEGENDS TO THE FIGURE

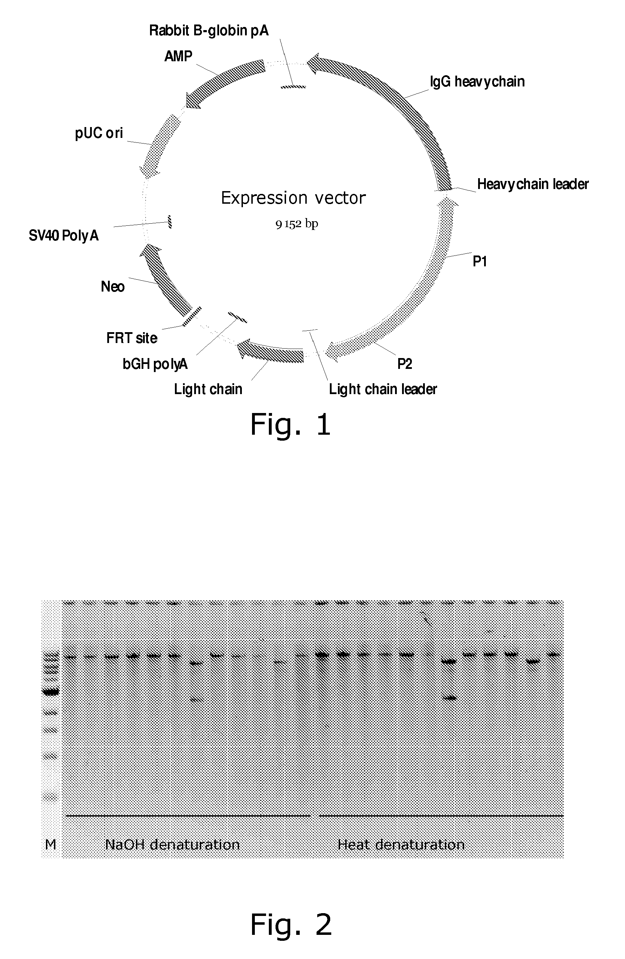

Fig. 1: Map showing the type of antibody expression plasmids used in the Examples. Two promoters (P1 and P2) are driving the expression of antibody heavy and light chains, respectively. Leader peptides are present for efficient secretion of both heavy and light chain as well as polyadenylation regions - rabbit β-globin poly A for the heavy chain and bovine growth hormone (bGH polyA) for the light chain. Also present are the following elements: β - lactamase for ampicillin resistance (AMP), *E. coli* origin of replication (pUCori) and a cassette for site-specific integration into FRT sites located in the genome of mammalian cells encoding the bacterial Neomycin resistance gene (FRT site, Neo, SV40 polyA).
Fig. 2: Result of TempliPhi® amplification using two different denaturation schemes. The lanes underlined on the left indicate amplification products obtained from NaOH denatured material, whereas the lanes underlined on the right indicated amplification products obtained from heat denatured material. The lane marked M is a molecular weight marker.
Fig. 3: The inventive setup for screening of positive clones in a eukaryotic system using the presently claimed technology.
Fig. 4: Tetanus-specific FMAT assay. The percentages of positive wells in a triplicate CHO-transfection are shown.

### Detailed disclosure of the invention

For the purposes of the present specification and claims, the following terms and expressions are explained in more detail:

An "**E**xpression **P**roduct with at least one **P**redetermined **F**unctionality", also termed an EPPF, is in the present context the product of eukaryotic expression of a nucleic acid, where said expression product exhibits a functionality which can be assayed for. Typically, the EPPF is a polypeptide or a protein, but it may also be a biologically active RNA molecule such as microRNA (miRNA), small interfering RNA (siRNA) or a ribozyme.

A "vector nucleic acid" is a nucleic acid constituting or forming part of a vector, such as a cloning vector or an expression vector. The vector nucleic acids tested in the present invention are those which include genetic elements which will allow an eukarotic cell to express the vector nucleic acid, whereby an EPPF is the expression product.

If a sample includes "one single vector nucleic acid species", this means that essentially all vector nucleic acids present in the sample have the same nucleotide sequence.

To "amplify nucleic acids in a molecular amplification procedure" means that one single amino acid sequence (the "template") is amplified in an in vitro system where there is no necessity for living cells. Typical amplification procedures known in the art are polymerase chain reaction (PCR) and nucleic acid sequence based amplification (NASBA), but for the purposes of the present invention a number of other methods are preferred, cf. below.

"Rolling circle amplification" is a molecular amplification process, where circular DNA is amplified

An "amplification product" is the nucleic acid being the result of a molecular amplification procedure.

A "concatemer" of a vector nucleic acid species is a molecule which includes multiple repeats of the vector nucleic acid species which are linked end-to-end.

"Transfection" is a term generally used to denote a method for introduction of nucleic acid into a host cell. Transfection may be transient meaning that the transfected DNA is not introduced into the host cell genome and therefore will not be stably passed on from parent to daughter cells during mitotic cell division. Transfection may also be "stable" or "permanent", meaning that the transfected nucleic acids are introduced into the host cell genome and stably passed on from parent to daughter cells during mitotic cell division.

Transfection of "a separate population of cells" with an amplification product means that a distinguishable population or subset of host cells are transfected with the same amplification product. This can e.g. be done by maintaining host cells in separate compartments, where the cells of each compartment are only transfected with identical amplification products.

"A eukaryotic cell" is a cell comprising a nucleus. Examples are plant cells and fungal cells (including filamentous fungi and yeast), protozoans, animal cells from invertebrates such as insects and vertebrates such as mammals, fish and birds.

"Culturing" denotes the technology of maintaining cells in vitro, so as to sustain their metabolism and allow them to proliferate and/or express genes (autologous as well as heterologous). In the context of the present invention, culturing is typically performed using conditions which, for the host cell of choice, facilitates expression of genes - this may e.g. entail use of specialized culture media and physical culture conditions known to the skilled person, but also of expression inducers (e.g. to induce inducible promoters controlling recombinant genes introduced into the host cells).

A "ligand" is a molecule which exhibits binding to a specific binding partner (such as a receptor). An "antigen" is a type of ligand which specifically binds an antibody or a T-cell receptor.

The term "antibody" describes a functional component of serum and is often referred to either as a collection of molecules (antibodies or immunoglobulins, fragments, etc) or as one molecule (the antibody molecule or immunoglobulin molecule). An antibody molecule is capable of binding to or reacting with a specific antigenic determinant (the antigen or the antigenic epitope), which in turn may lead to induction of immunological effector mechanisms. An individual antibody molecule is usually regarded as monospecific, and a composition of antibody molecules may be monoclonal (i.e., consisting of identical antibody molecules) or polyclonal (i.e., consisting of different antibody molecules reacting with the same or different epitopes on the same antigen or on distinct, different antigens). The distinct and different antibody molecules constituting a polyclonal antibody may be termed "members". Each antibody molecule has a unique structure that enables it to bind specifically to its corresponding antigen, and all natural antibody molecules have the same overall basic structure of two identical light chains and two identical heavy chains. Antibodies are also known collectively as immunoglobulins. The terms antibody or antibodies as used herein are used in the broadest sense and cover intact antibodies, non-human e.g. murine or rabbit etc., chimeric, humanized, fully human and single chain antibodies, as well as binding fragments of antibodies, such as Fab, Fv fragments or scFv fragments, as well as multimeric forms such as dimeric IgA molecules or pentavalent IgM.

The term "polyclonal antibody" describes a composition of different (diverse) antibody molecules which are capable of binding to or reacting with several different specific antigenic determinants on the same or on different antigens. Usually, the variability of a polyclonal antibody is located in the so-called variable regions of the polyclonal antibody, in particular in the CDR regions. In the present invention a mixture of two or more polyclonal antibodies (a polycomposition) is preferably produced in one pot from a polyclonal polycomposition cell line, which is produced from two or more parental polyclonal cell lines each expressing antibody molecules which are capable of binding to a distinct target, but it may also be a mixture of two or more polyclonal antibodies produced separately. A mixture of monoclonal antibodies providing the same antigen/epitope coverage as a polyclonal antibody of the present invention will be considered as an equivalent of a polyclonal antibody. When stating that a member of a polyclonal antibody binds to an antigen, it is herein meant to be binding with a binding constant below at least 1µM, and below 100 nM, preferably below 10 nM, even more preferred below 1 nM.

The term "recombinant antibody" is used to describe an antibody molecule or several molecules that is/are expressed from a cell or cell line transfected with an expression vector comprising the coding sequence of the antibody which is not naturally associated with the cell. If the "antibody molecules" in a "recombinant antibody" are diverse or different, the term "recombinant polyclonal antibody" applies in accordance with the definition of a "polyclonal antibody".

The term "immunoglobulin" commonly is used as a collective designation of the mixture of antibodies found in blood or serum, but may also be used to designate a mixture of antibodies derived from other sources, or may be used synonymous for the term "antibody".

The classes of human antibody molecules are: IgA, IgD, IgE, IgG and IgM. Members of each class are said to be of the same isotype. IgA and IgG isotypes are further subdivided into subtypes. The subtype(s) of IgA and IgG commonly refers to IgA₁, IgA₂ and IgG₁, IgG₂, IgG₃ and IgG₄, respectively.

The terms "cognate V_{H} and V_{L} coding pair" or "cognate pairs of V_{H} and V_{L} sequences" describes an original pair of V_{H} and V_{L} coding sequences contained within or derived from the same cell. Thus, a cognate V_{H} and V_{L} pair represents the V_{H} and V_{L} pairing originally present in the donor from which such a cell is derived. The term "an antibody expressed from a V_{H} and V_{L} coding pair" indicates that an antibody or an antibody fragment is produced from a vector, plasmid or similar containing the V_{H} and V_{L} coding sequences. When a cognate V_{H} and V_{L} coding pair is expressed, either as a complete antibody or as a stable fragment thereof, they preserve the binding affinity and specificity of the antibody originally expressed from the cell they are derived from. A composition of cognate pairs is also termed a repertoire of cognate pairs, and may be kept individually or pooled.

The term "recombinant polyclonal cell line" or "polyclonal cell line" refers to a mixture/population of protein-expressing cells that are transfected or transduced with a repertoire of variant nucleic acid sequences (e.g. a repertoire of antibody-encoding nucleic acid sequences), such that the individual cells, which together constitute the recombinant polyclonal cell line, each carry at least one transcriptionally active copy of a distinct nucleic acid sequence of interest, which encodes one member of the recombinant polyclonal antibody of interest. In one embodiment of the invention, only a single copy of a distinct nucleic acid sequence is integrated at a specific site in the genome of each of the cells. The cells constituting the recombinant polyclonal cell line are selected for their ability to retain the integrated copy of the distinct nucleic acid sequence of interest, for example by antibiotic selection. Cells which can constitute such a polyclonal cell line can be for example bacteria, fungi, eukaryotic cells, such as yeast, insect cells, plant cells or mammalian cells, especially immortal mammalian cell lines such as CHO cells, COS cells, BHK cells, myeloma cells (e.g., Sp2/0 cells, NSO, YB2/0), NIH 3T3, and immortalized human cells, such as HeLa cells, HEK 293 cells, or PER.C6.

The terms "sequences encoding V_{H} and V_{L} pairs" or "V_{H} and V_{L} encoding sequence pairs" indicate nucleic acid molecules, where each molecule comprise a sequence that code for the expression of a variable heavy chain and a variable light chain, such that these can be expressed as a pair from the nucleic acid molecule if suitable promoter and/or IRES regions are present and operably linked to the sequences. The nucleic acid molecule may also code for part of the constant regions or the complete constant region of the heavy chain and/or the light chain, allowing for the expression of a Fab fragment, a full-length antibody or other antibody fragments if suitable promoter and/or IRES regions are present and operably linked to the sequences.

The term "polypeptide" is in the present context intended to mean molecules comprising multiple amino acids covalently linked via peptide bonds, and the term encompasses both short peptides of from 2 to 10 amino acid residues, oligopeptides of from 11 to 100 amino acid residues, and poly-peptides of more than 100 amino acid residues. Furthermore, the term is also intended to include proteins, i.e. functional biomolecules comprising at least one polypeptide; when comprising at least two polypeptides, these may form complexes, be covalently linked, or may be non-covalently linked. The polypeptide(s) in a protein can be glycosylated and/or lipidated and/or acetylated and/or phosphorylated and/or comprise prosthetic groups. Thus the term includes enzymes, antibodies, antigens, transcription factors, binding proteins e.g. DNA binding proteins, or protein domains or fragments of proteins or any other amino acid based material.

The term "polyamino acid" denotes a molecule constituted by at least 3 covalently linked amino acid residues.

The term "subsequence" means any consecutive stretch of at least 3 amino acids or, when relevant, of at least 3 nucleotides, derived directly from a naturally occurring complete amino acid sequence or nucleic acid sequence, respectively.

A "functional part" of a (bio)molecule such as a DNA polymerase is in the present context intended to mean the part of the molecule which is responsible for at least one of the biochemical or physiological effects exerted by the molecule. It is well-known in the art that many enzymes and other effector molecules have an active site which is responsible for the effects exerted by the molecule in question. Other parts of the molecule may serve a stabilizing or solubility enhancing purpose and can therefore be left out if these purposes are not of relevance in the context of its desired function. An example of such a functional part of a biomolecule is the Klenow fragment of DNA polymerase I.

When using the expression "derived products" herein, this indicates that the products in question (e.g. an amplification product, a vector or a host cell, all prepared from a starting nucleic acid) all share the feature that they at least include the coding sequence for a (putative) EPPF.

### Detailed discussion of the preferred embodiments

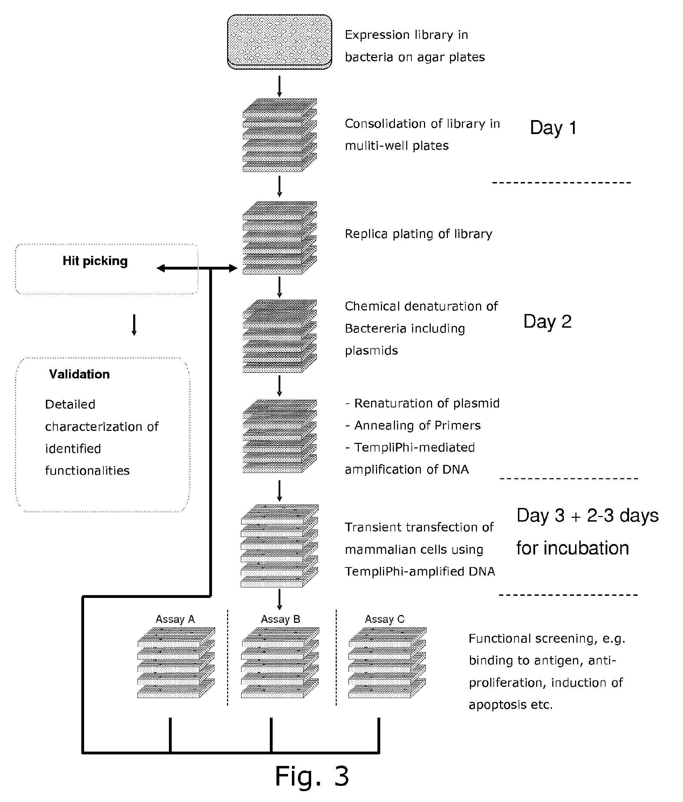

The present invention allows for simple, convenient and fast identification of vectors (typically derived from libraries of vectors and/or from bacterial cells transfected with nucleic acids of which at least some are expected to encode the EPPF) which encode and can effect expression of a functional expression product in eukaryotic cells. As shown in Fig 3 (method of the invention), the number of steps and the time needed to obtain confirmatory results of the usefulness of a given vector is reduced in the method of the invention, i.a. because the step of obtaining sufficient material for functional screening is much simpler and less resource consuming in the method of the invention. A particular advantage of the methods of the invention is that they can be subjected to automation using standard laboratory robots.

Even though the focus has been put on demonstrating the inventive concept by identifying antibodies with specific binding and neutralization characteristics, the functionalities which can be tested by the method of the invention are numerous.

The invention provides that it is possible to identify EPPFs, where the functionality is selected from the group consisting of
- exertion of a physical effect by the expression product - this can e.g. be emission of fluorescence or luminescence, or absorbance of light or other electromagnetic radiation;
- binding of the expression product to a ligand or antigen - typical examples are binding of receptor molecules/antibodies;
- exertion by the expression product of catalytic activity, such as enzymatic activity;
- susceptibility of the expression product to catalytic activity, such as enzymatic activity;
- facilitation by the expression product of altered transport of an agent across a biological membrane;
- facilitation by the expression product of altered translocation of an agent in the intracellular compartment;
- influence by the expression product on expression of at least one gene in a population of eukaroytic cells - i.e. the function of the expression product as an expression regulator (e.g. on the transcription level) can be assayed;
- influence by the expression product on the growth or metabolism of a population of eukaryotic cells - convenient in cases where libraries encoding mutated versions of a growth regulator is screened;
- influence by the expression product on target cells - it is e.g. possible to test for the expression product's effects on proliferation, anti-proliferation, differentiation, apoptosis, metabolism, sensitivity to drug, all of relevance when screening for expression products which could e.g. function as anti-cancer drugs;
- influence by the expression product on a pathogenic agent - it is possible to assay for virus neutralisation, binding to virus, killing of virus, and likewise it is possible to assay for binding to bacteria, killing of bacteria, etc.; and
- influence by the expression product on secondary immune effects.

For some of these assays, it may be advantageous to partly purify the expression product prior to performing the assay. In the case of secreted expression products, such as secreted proteins, this may be done in a high throughput manner by using a solid surface with an affinity for the expression produce, for example beads, columns, sticks or other solid surfaces. The secreted proteins can subsequently be eluted from the surface. High throughput purification using columns adapted for use in multiwell dishes is also a possibility.

All of these above-mentioned functionalities are exemplary - the method of the invention does not depend on the precise choice of assay or test utilised in steps e) or E) (depending on the aspect of the invention), which can be any test/assay known to the skilled person to determine the presence of a functional expression product in or from a cell.

The expression product whose presence or activity is identified/quantified, can be any expression product and is typically selected from an RNA (such as a ribosome, a miRNA or a siRNA), a peptide, an oligopeptide, a polypeptide, a monomeric protein and a multimeric protein. The invention also allows for identification of protein expression products which include characteristic glycosylation patterns or other posttranslational modifications.

As demonstrated herein, one very convenient feature of the present invention is the fact that culture supernatants from step d) or D) can be assayed directly in step e) or E), respectively. Of course, this requires that the expression product is secreted by the eukaryotic cells - but for many expression products this is readily accomplished by incorporating genetic information encoding suitable secretion signals so as to allow export from the eukaryotic cells. Hence, the method of the invention is conveniently one, wherein testing in step e) or E) involves an assay for the presence and/or activity of the EPPF in the culture supernatant. This provides the advantage that the expression product can be tested for a predetermined functionality without the presence of the cell having expressed the product. This widens the possibilities for carrying out functional assays compared to the known display systems, in which the phenotype (expression product) is linked directly to the genotype (the cell).

In the practice of the first embodiment of the invention it is of importance that the test in step e) can be readily related to one of the samples of vector nucleic acids in step a). Termed otherwise, in the practice of the present invention it is convenient that steps a)-e) of the first aspect of the invention ensure that a population of eukaryotic cells wherein the EPPF is found to be present in step e) is unambiguously derived from one vector nucleic acid species of step a).

It is, however, not always a prerequisite that it is possible to correlate the sample to a "positive hit" from the test step. Since the precise sequence information exists with respect to the obligate genetic elements of the expression vector cassette, it is possible to excise or amplify and sequence the coding sequence for the expression product from the expression host and instead determine the coding sequence of the gene encoding the EPPF.

Hence, the present invention also envisages the above-discussed second aspect of the invention in the form of an alternative method for identifying and/or isolating vector nucleic acids, which each encodes an expression product with at least one predetermined functionality (EPPF) and which includes genetic elements sufficient to express said EPPF in a eukaryotic cell, the method comprising
A) providing a plurality of nucleic acids each including a nucleotide sequence which putatively encodes an EPPF,
B) amplifying, from each member of the plurality of nucleic acids, at least the nucleotide sequence which putatively encodes the EPPF in a molecular amplification procedure to produce amplification products being concatemers each including said nucleotide sequence in operable linkage with genetic elements sufficient to express said nucleotide sequence in a eukaryotic cell and,
C) transfecting each of said amplification products into eukaryotic cells,
D) culturing the eukaryotic cells under conditions which facilitate expression of the amplification products,
E) subsequently testing for the presence of said EPPF in the eukaryotic cells, and
F) identifying and/or isolating a vector including the nucleotide sequence encoding an EPPF found to be present in step E).

It will be apparent that the coding sequence for the EPPF found in step E) can be retrieved by excising or amplifying and optionally sequencing it from the amplification product present in the eukaryotic cell. This can for example be done if the EPPF is located inside the cell or on the surface of the cell, so that the phenotype (EPPF) and genotype (expression vector) is linked directly.

The vector identified may of course be one single functional expressible unit of the concatemer amplification product, but alternatively the vector nucleic acids may be produced or identified by simply combining the coding sequence for the EPPF found in step E) with remaining necessary genetic elements sufficient to effect expression of the EPPF in a eukaryotic cell. Hence, the coding sequence for the EPPF can subsequently be combined with any convenient expression cassette useful in eukaryotic cells.

However, preferred embodiments of the invention are those, wherein the vector nucleic acid species in step a) of the first embodiment as well as their derived products are physically separated from each other in each step, or wherein the vector nucleic acids in step a) as well as their derived products are uniquely labelled - both these modes of the invention ensure that it is unproblematic to identify the vector nucleic acid in step a) directly on the basis of the test result in step e) without any need for further steps of isolating and/or sequencing material from the eukaryotic cells used in the test steps. Hence, it is preferred that the first aspect of the invention entails that the plurality of samples and/or vector nucleic acids in step a) as well as their derived products are arrayed and/or catalogued so as to facilitate the identification in step f). A convenient way to physically separate vector nucleic acid species is the use of standard multiwell dishes in 96 or 384 well format.

In practical embodiments of the present invention, the vector nucleic acids in step a) or the nucleic acids in step A) are selected from a circular DNA such as a bacterial plasmid or a bacterial chromosome. Many libraries of nucleic acids are prepared using cloning vectors in this format, and traditionally these cloning vectors are then separated from other DNA in several time-consuming steps before they may be used for testing in relevant eukaryotic cells. The present invention facilitates this particular part of an expression cloning process, because the bacterial cells containing the DNA can be directly subjected to denaturing conditions (e.g. heat or base denaturation - the latter being a generally preferred feature of the methods of the invention) and subsequently be subjected directly to molecular amplification. The ability to use base denaturation makes the method more applicable to automation as it is easier to control the addition of a base than to control temperature precisely.

It is preferred that the molecular amplification procedure comprises rolling circle amplification. Rolling circle amplification produces concatemers of the template nucleic acid molecule, a feature which is generally preferred in the present invention.

Polymerases useful in the present methods include those that will initiate 5'-3' polymerization. The polymerase should also displace the polymerized strand downstream from the initiation site, and, importantly, should also lack any 5'→3' exonuclease activity. Polymerases, such as the Klenow fragment of DNA polymerase I and the exonuclease deficient Klenow fragment of DNA polymerase I and a similar fragment from the Bst polymerase (Bio-Rad, Richmond, Calif.) are useful. SEQUENASE 1.0 and SEQUENASE 2.0 (US Biochemical), T5 DNA polymerase, Vent_{R}(exo-) (New England Biolabs), ThermoPhi (Prokaria, Iceland), and Phi29 DNA polymerases (TempliPhi®, GE Healthcare) also work. Alternatively, it would also be possible to PCR amplify the entire expression cassette.

Especially preferred polymerases are bacteriophage phi29 DNA polymerase or a functionally equivalent polymerase, such as truncated phi29 polymerase, and a mutant of phi29 polymerase having at least 80% sequence identity with phi29 or with a functional truncate thereof. This sequence identity may be higher, e.g. at least 81, 82, 83, 84, 85, 86, 87, 88, 89, 90, 91, 92, 93, 94, 95, 96, 97, 98, or 99%. Sequence identity between a reference amino acid sequence and a variant amino acid sequences is performed by aligning the sequences using the default settings of Clustal W (1.82). The number of fully conserved residues is counted and divided by the total number of residues in the reference sequence.

An additional feature of rolling circle amplification is that it does not require temperature cycling, i.e. it is isothermal (which is also a preferred feature of the methods of the invention). Many amplification methods (e.g. traditional PCR) require temperature cycling in order to dissociate the target from the synthesized strand. In rolling circle amplification a single temperature may be employed after denaturation has occurred. The absence of temperature cycling means that the method is more simple and robust and easier to automate. In addition, proper temperature should support efficient enzyme activity, determined mainly by the temperature optimum of the enzyme, typically between 15 and 50°C such as between 20 and 45°C or between 15 and 30°C. For the TempliPhi enzyme, a temperature around 30°C has been found effective. The important consideration in this context is to ensure that denaturation of the polymerases and nucleic acid is avoided.

The testing steps in the method of the invention can be designed according to any protocol known to the person skilled in the art. They may comprise test for presence or activity of the EPPF in the culture medium (as a secretion product or a shed membrane protein), EPPF present on the surface of the eukaryotic cells and/or intracellular EPPF. Preferably, the culture medium is tested for the presence or activity of the EPPF. The present invention enables testing of culture medium for e.g. virus neutralization or antiproliferative activity as opposed to the known display systems.

Preferably, the EPPF is an antibody, antibody fragment, or a synthetic or semi-synthetic antibody or antibody analogue which binds a predetermined antigen. As shown in the present set of Examples, antibodies are readily expressed and secreted from cells transfected with TempliPhi amplifed DNA, allowing for the detection of the antibodies directly in the culture supernatant from the transfected cells. The present invention hence facilitates screening of large antibody encoding DNA libraries so as to allow identification of interesting/useful antibody species which fulfill predetermined binding and other functional criteria in a relevant biological system.

Having identified/isolated a nucleic adid encoding an EPPF, it is possible to utilise this DNA for recombinant production of the EPPF in eukaryotic cells - the methods in the two first aspects of the invention having confirmed that the nucleic acid is expressible in a eukaryotic system. Hence, the invention also pertains to a method for producing an EPPF in eukaryotic cells, the method comprising identifying a vector nucleic acid by means of the methods of the invention, and subsequently transfecting or transducing a suitable eukaryotic host cell with the vector from where the vector nucleic acid is derived, culturing the transfected or transduced eukaryotic cell under conditions which facilitate expression of the EPPF encoding nucleic acid and recovering the EPPF from the culture. Preferably, the EPPF is an antibody, antibody fragment or antibody analogue.

The plurality of vector nucleic acid species encoding an EPPF are preferably derived through a multiplex molecular amplification procedure using separate templates derived from isolated single cells, the molecular amplification comprising generating cognate pairs of variable region encoding sequences through linking of nucleotide sequences encoding variable region each cognate pair being derived from one isolated single cell. Methods for cognate pairing of variable region encoding sequences (the Symplex^{™} technology) are described in WO 2005/042774. The Symplex^{™} technology provides arrayed or catalogued vectors encoding cognate pairs of V_{H} and V_{L} chains in a format suitable for screening using the methods of the invention.

Such recombinant production is accomplished utilizing methods generally known to the person of skill in the art - numerous useful methods are e.g. set forth in Sambrook J et al. 2000. Molecular Cloning: A Laboratory Manual (Third Edition) and in the corresponding on-line manual: www.MolecularCloning.com.

### Alternative use of the present invention

In addition to Symplex^{™} antibody repertoire screening, TempliPhi amplified DNA is applicable to other approaches that operate cDNA libraries or *in vitro* generated composition of protein variants. Especially in situations that requires a catalogued array of the tested clones in order for back tracing of positive reactions during the screening. One example may be Alanine scanning of a secreted protein for activity knock outs in order to identify amino acid residues important for the biological interaction or reaction and thereby the assayed activity. If the protein of interest is a growth stimulating protein a library may be constructed by error prone PCR or alternative mutagenesis technologies [Ling MM and Robinson BH. Approaches to DNA mutagenesis: an overview. Anal Biochem, 1997;254:157-178; Wang TW, Zhu H, Ma XY, Zhang T, Ma YS and Wei DZ. Mutant library construction in directed molecular evolution: casting a wider net. Mol Biotechnol, 2006;34:55-68] and the produced library arrayed and tested for function. Screening for clones with loss-of-activity can be performed and essential residues identified. In another scenario where the proteins of interest induce apoptosis or inhibit the cell growth of the target cell line and the screening parameter is lack of cell proliferation or cell death a catalogued library will be required. The present invention using the TempliPhi amplified DNA of the libraries will allow efficient screening of arrayed catalogued cDNA libraries for proteins with such activities. Similarly, the invention will be useful for screening mutated libraries for improved function. One example could be screening a library where an antibody with a desired activity is mutated (error-prone PCR or alternative mutagenesis procedures) in the CDR regions for mutants with improved function, such as improved affinity and/or specificity.

### EXAMPLES

### EXAMPLE 1

### TempliPhi® reactions using 2 different DNA denaturing schemes

### Preparation of TempliPhi DNA:

The 12 plasmids (Figure 1) used in the following were derived from a project aiming at providing an anti-tetanus polyclonal antibody. One of these plasmids (no. 11, 2-B9) was without a promoter fragment and accordingly smaller in size.

The reactions were performed with the TempliPhi® amplification kit, Amersham Biosciences cat. no. 25-6400-10.

Bacterial colonies containing the 12 plasmids were picked into 384-well plates containing 150 µl 2 x YT medium containing 100 µg/ml carbenicillin and shaken overnight at 37°C.

### Heat denaturation:

1 µl of bacterial culture was mixed with 25 µl of H₂O and 0.5 µl of the resulting mixture was subsequently mixed with 5 µl of Sample Buffer (from the TempliPhi amplification kit). Denaturation of bacterial DNA including plasmid DNA was performed for 3 minutes at 95°C. 5 µl of reaction buffer (from the TempliPhi amplification kit) and 0.2 µl of TempliPhi enzyme (the bacteriophage phi29 DNA polymerase) was added and amplification was performed at 30°C overnight.

### Denaturation with NaOH:

1 µl of bacterial culture was mixed with 25 µl 0.1 M NaOH and incubated for 5 minutes at room temperature. To 0.5 µl of this chemically denaturated DNA was then added 5 µl of Sample Buffer, 5 µl of Reaction Buffer and 0.2 µl of TempliPhi enzyme and amplification was performed at 30°C overnight.

### Evaluation:

All amplifications were denatured at 65°C for 10 minutes followed by linearization of DNA by incubation with the DNA endonuclease PvuI and analysis by agarose gel electrophoresis.

The gel analyses demonstrated that the amplification works equally well using the two denaturation schemes (Fig. 2). One plasmid (no. 7) has an internal PvuI site in the antibody variable regions, while the plasmid without promoter fragment (no. 11) is clearly smaller in size. It was concluded that chemically mediated denaturation works as efficiently as heat denaturation.

### EXAMPLE 2

TempliPhi DNA representing the 12 different plasmids (Figure 1) was prepared as described in Example 1 using heat denaturation. After amplification the reaction mixtures were diluted with 4 volumes of H₂O. CHO Flp-In cells (Invitrogen) were seeded into 384-well plates (Applied Biosystems, cat.no. 4307723) in a density of 2000 cells per well in 20 µl of Ham-F12 medium containing 10% fetal calf serum. A total of four transfections were performed in parallel with each TempliPhi preparation.

Transfection was performed the following day (the day after seeding). Transfection mixes were prepared as follows:
- 1.5 µl FuGENE 6 (Roche) and 28.5 µl Dulbecco-modified Eagle's medium (DMEM) was incubated for 5 minutes at room temperature
- FuGENE-DMEM was mixed with 10 µl of TempliPhi amplified DNA and incubated at room temperature for 15 minutes (transfection mix)
- 2 µl of each FuGENE-DMEM-DNA mix was added to each well in the 384-well plate containing cells and the cells were cultured for 48 hours

The cell supernatants were analysed for the presence of tetanus specific antibodies by a direct binding assay utilising the FMAT technology (fluorometric microvolume assay technology). For these FMAT assays the following reagents were used:
- FACS buffer (PBS; 1% bovine serum albumin (BSA))
- polystyrene-beads coated with tetanus toxoid (Statens Serum Institut, Denmark) suspended in FACS buffer
- polystyrene-beads coated with goat anti-human kappa antibody (Serotec STAR100) suspended in FACS buffer
- fluorescent detection antibody: goat anti-human IgG conjugated with Alexa Fluor 647 (Molecular Probes cat. no. A-21445)

After 48 hrs supernatants were aspirated and mixed with fluorescent detection antibody and coated (tetanus toxoid or anti-kappa antibody) beads. The final dilution of the supernatants was 1:10. After 3 hours of incubation fluorescence was read on an ABI8200 cellular detection system. 50 fluorescent events served as cut-off value, below which fluorescent readings are given as 0. The results are shown in table 1.

**Table 1: FMAT assay on supernatants after TempliPhi transfection. Supernatants were tested in a 1:10 dilution against tetanus toxoid or anti-kappa coated beads. The plasmid without promoter is designated 2-B9. Results are shown as the mean of 4 determinations.**

| Clone designation | Fluorescence reading, tetanus toxoid beads | Fluorescence reading, anti-kappa beads |
|---|---|---|
| 1-A7 | 3003 | 679 |
| 1-A10 | 397 | 128 |
| 1-B6 | 0 | 278 |
| 1-F4 | 0 | 0 |
| 1-H4 | 1866 | 1065 |
| 2-B9 | 0 | 0 |
| 2-C3 | 0 | 0 |
| 2-G9 | 0 | 0 |
| 2-H10 | 0 | 292 |
| 3-H6 | 983 | 569 |
| 4-H5 | 0 | 0 |
| 4-H6 | 1018 | 396 |

7 out of 12 amplification products provided a positive signal for IgG. The promoterless plasmid was negative as expected. In the remaining 4 transfections where no signal is seen, values may be positive but below the detection limit. Five of the 12 TempliPhi preparations exhibited binding to tetanus toxoid-coated beads and all of these were positive for IgG.

Supernatants from TempliPhi transfection performed as described above were analyzed for human IgG content in a sandwich ELISA using anti-human kappa antibody for catching and peroxidase-conjugated anti-human IgG for detection. IgG concentrations in FMAT-IgG-positive wells had a mean value of 70 ng/ml.

### EXAMPLE 3

Automated procedures for TempliPhi reaction set up, transfection and screening in 384-well format were developed using an Aquarius 96 (TECAN) liquid handler. *E. coli* harbouring a plasmid library of mammalian expression vectors encoding anti-tetanus specific antibodies (Figure 1) with a frequency of specific anti-tetanus antibodies of approx. 10% was used as template.

### Automated preparation of TempliPhi DNA:

25 µl 0.1 M NaOH was dispensed in each well in a ScreenMate 384 deep well (Matrix). 0.5 µl overnight culture from random single *E. coli* colonies, derived from the anti-tetanus library, was dispensed and mixed by aspirating and dispensing 3 times in each well of the plate. The cultures were prepared as in Example 1 in a Screen Mate 384-well deep well plate, leaving row 1 empty. Thus, 368 colonies were inoculated in total. The mix of *E. coli* culture and 0.1 M NaOH was incubated for 15 minutes

10 µl TempliPhi reaction mix (5 µl Enzyme buffer + 5 µl Denaturing buffer) of TempliPhi (TempliPhi kit 25-6400-01) was dispensed in an AB-1111 384-well plate. 0.5 µl of the E.coli/NaOH suspension was transferred to the plate with TempliPhi reaction mix and mixed by aspirating and dispensing 3 times. The plate was sealed and incubated at 30°C overnight.

### Automated transfection of CHO-cells:

CHO-cells were prepared and seeded in three 384-well plates as in Example 2. Transfection was performed in triplicate the following day by preparing a mix of FuGene and DMEM in a ratio of 1:19.6 µl of the FuGene/DMEM mix is dispensed in each well in an AB-1111 384-well plate (ABgene). 2 µl of the TempliPhi amplified DNA was transferred from the TempliPhi plate above to the FuGene/DMEM mix plate. The reaction was mixed by aspirating and dispensing 3 times and was incubated for 15 min at room temperature. 2 µl of the FuGene/DMEM/DNA mix was then transferred to each of the CHO-cell plate wells. A gentle mixing was performed by aspirating and dispensing. The transfected CHO-cell plates were incubated for 2 days at 37°C in a 5% CO₂ atmosphere.

### Harvest of supernatants:

After 2 days the CHO-cell supernatants were harvested by first adding 20 µl PBS + 1% BSA to the transfected cells and then transferring the diluted supernatant to an identical but empty 384-well plate. The supernatant was frozen at -20°C before further analysis.

### FMAT assay of TT-specific antibodies:

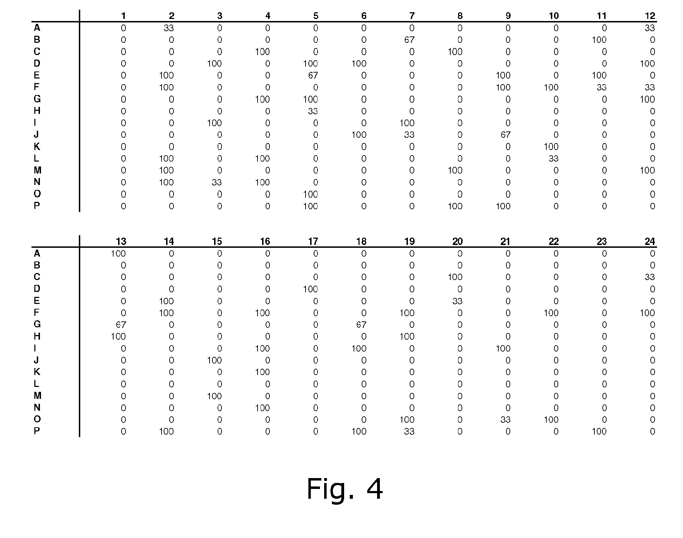

The 384-well plates with supernatants were thawed and further diluted by transferring 30 µl supernatant and adding 45 µl PBS + 1% BSA. The final dilution of the supernatants was 1:5. The supernatant were analysed by FMAT using beads coated with tetanus toxoid as in example 2. All wells that gave rise to a positive FMAT signal (using 50 counts as cut off as in example 2) were scored as positive (Cf. Fig. 4). 76% of the positive wells were identical in all three plates, 17% of the wells were scored positive in two out of three plates and 7% of the wells were scored positive in one out of three plates. In all, 71 positive wells were identified if all positive well-positions are counted in the three plates.

### EXAMPLE 4

### Screening for binding to EGFR extracellular domain

The method of the invention was subsequently tested using a murine repertoire of linked V_{H} and V_{L} coding pairs from antibodies reactive with human EGFR, spliced to the human kappa constant coding region. Briefly, mice were immunised with human EFGR or the extracellular domain of human EGFR, B-cells were harvested and Symplex^{™} ((Meijer et al, 2006, J. Mol. Biol, 358:764-772; WO 2005/042774) linking of cognate V_{H} and V_{L} sequences was performed. The PCR fragments were subsequently transferred to an expression vector similar to the one shown in Figure 1.

Production and culture of transfected CHO cells as well as harvest of supernatants were thereafter performed as described in Example 3.

In general, the screening of the supernatants was made as a two step procedure. The antibody-libraries containing supernatants were screened for reactivity to recombinant EGFR protein in ELISA after which FMAT (FLISA) was used as a cell based approach, with the NR6wtEGFR cell line, for detection of EGFR-antibodies binding to cell-surface expressed EGFR. For some libraries, the ELISA was performed with recombinant EGFR representing the extracellular domain of the EGFR only.

Briefly for the ELISA, Nunc maxisorb plates (cat no 464718) were coated with 1 µg/ml protein (in house produced), diluted in PBS at 4°C overnight. Prior to blocking in 50 µl 2%-Milk-PBS-T the plates were washed once with PBS + 0.05 % Tween 20 (PBS-T). The plates were washed once with PBS-T, 20 µl of 2%- milk-PBS-T and 5 µl supernatants were added and incubated for 1.5 hour at room temparature after which the plates were washed once with PBS-T 20 µl per well. Secondary antibody (HRP-Goat-anti-human IgG, Jackson, cat. no. 109-035-097) diluted 1:10000 in 2% milk-PBS-T was added to detect the antibodies present in the supernatant and incubated for 1 hour at room remperature. The plates were washed once in PBS-T before addition of 25 µl substrate (Kem-en-tec Diagnostics, cat. no. 4390) that was incubated for 5 minutes before 25 µl 1 M H₂SO₄was added to stop the reaction. Specific signal was detected as absorbance at 450 nm.

For the cell based FMAT detection of anti-EGFR antibodies, SKBR-3 (ATCC #HTB-30) or NR6wtEGFR (Welsh et al, 1991, J Cell Biol, 114, 3, 533-543) cells were kept in growth medium as described. The cells were counted and diluted to 1.25 x 10⁵ cells/ml with the alexa-647 conjugated goat-anti-human IgG (H-L) antibody (Molecular probes No. A21445, lot no. 34686A) diluted 1:40,000. A total of 20 µl of this suspension was transferred to 384 well clear bottom Nunc plates. Subsequently 10 µl transfection supernatant was added to the cells. The FMAT signal from the reaction was measured after 6-10 hour of incubation.

The data from the screening indicates that 221 (4.8%) of the total clones were positive in the ELISA. 93 (2.0%) of those clones were also positive in FMAT. In total 220 (4.8%) of the clones were positive in the FMAT and among those 127 (220-93) uniquely positive for the cell surface antigen. One library was screened in a similar fashion, but since the immunization procedure was made to generate antibodies specific for the deletion mutant EGFR receptor EGFRvIII, the ELISA screenings included assays to detect both wild-type EGFR and EGFRvIII. Seven clones were identified to be specific for the EGFRvIII in the ELISA and interestingly those clones were negative for staining of wtEGFR expressing cells in the FMAT. 13 clones were identified to be positive for the wtEGFR in FMAT and ELISA but not for the EGFRvIII, which were unique for this library. All the ELISA positive clones were selected for further analysis.

## Claims

1. A method for identifying and/or isolating vector nucleic acids, which each encodes an expression product with at least one predetermined functionality (EPPF) and which includes genetic elements sufficient to express said EPPF in a eukaryotic cell, the method comprising
a) providing a plurality of samples of vector nucleic acids, wherein each of said samples comprises one single vector nucleic acid species,
b) amplifying nucleic acids from each distinct sample in a molecular amplification procedure to produce amplification products being concatemers of said vector nucleic acid species,
c) transfecting each of said amplification products into a separate population of eukaryotic cells,
d) culturing each separate population of eukaryotic cells under conditions which facilitate expression of the amplification products,
e) subsequently testing for the presence of said EPPF in the cultures of eukaryotic cells, and
f) identifying and/or isolating the vector(s) of step (a) from which has/have been derived an EPPF found to be present in step e.

2. A method for identifying and/or isolating vector nucleic acids, which each encodes an expression product with at least one predetermined functionality (EPPF) and which includes genetic elements sufficient to express said EPPF in a eukaryotic cell, the method comprising
A) providing a plurality of nucleic acids each including a nucleotide sequence which putatively encodes an EPPF,
B) amplifying, from each member of the plurality of nucleic acids, at least the nucleotide sequence which putatively encodes the EPPF in a molecular amplification procedure to produce amplification products being concatemers each including said nucleotide sequence in operable linkage with genetic elements sufficient to express said nucleotide sequence in a eukaryotic cell and,
C) transfecting each of said amplification products into eukaryotic cells,
D) culturing the eukaryotic cells under conditions which facilitate expression of the amplification products,
E) subsequently testing for the presence of said EPPF in the eukaryotic cells, and
F) identifying and/or isolating a vector including the nucleotide sequence encoding an EPPF found to be present in step E).

3. The method according to claim 2, wherein the coding sequence for the EPPF found in step E) is determined by excising and sequencing it from the amplification product present in the eukaryotic cell.

4. The method according to claim 2 or 3, wherein the vector nucleic acids are identified by combining the coding sequence for the EPPF found in step E) with remaining necessary genetic elements sufficient to effect expression of the EPPF in a eukaryotic cell.

5. The method according to any one of the preceding claims, wherein said at least one predetermined functionality is selected from the group consisting of
- exertion of a physical effect by the expression product;
- binding of the expression product to a ligand or antigen;
- exertion by the expression product of catalytic activity, such as enzymatic activity;
- susceptibility of the expression product to catalytic activity, such as enzymatic activity;
- facilitation by the expression product of altered transport of an agent across a biological membrane;
- facilitation by the expression product of altered translocation of an agent in the intracellular compartment;
- influence by the expression product on expression of at least one gene in a population of eukaroytic cells;
- influence by the expression product on the growth or metabolism of a population of eukaryotic cells;
- influence by the expression product on target cells;
- influence by the expression product on a pathogenic agent; and
- influence by the expression product on secondary immune effects.

6. The method according to any one of the preceding claims, wherein the expression product is selected from an RNA, a peptide, an oligopeptide, a polypeptide, a monomeric protein and a multimeric protein.

7. The method according to any one of claims 1 and 5 and 6, insofar as these are dependent on claim 1, wherein testing in step e) involves an assay for the presence and/or activity of the EPPF in the culture supernatant.

8. The method according to any one of claims 1 and 5-7, insofar as these are dependent on claim 1, wherein steps a)-e) ensure that a population of eukaryotic cells wherein the EPPF is found to be present in step e) is unambiguously derived from one vector nucleic acid species of step a.

9. The method according to claim 8, wherein the vector nucleic acid species in step a) as well as their derived products, are physically separated from each other in each step, or wherein the vector nucleic acids in step a) as well as their derived products are uniquely labelled.

10. The method according to claim 8, wherein the plurality of samples and/or vector nucleic acids in step a) as well as their derived products are arrayed and/or catalogued so as to facilitate the identification in step f).

11. The method according to any one of the preceding claims, wherein the vector nucleic acids in step a) or the nucleic acids in step A) are selected from a circular DNA such as a bacterial plasmid or a bacterial chromosome.

12. The method according to any one of the preceding claims, wherein the molecular amplification procedure comprises rolling circle amplification.

13. The method according to claim 12, wherein the rolling circle amplification utilises a strand-displacing DNA polymerase which substantially lacks 5'→3' exonuclease activity.

14. The method according to claim 12 or 13, wherein the strand-displacing DNA polymerase is selected from the Klenow fragment of DNA polymerase I, the exonuclease deficient Klenow fragment of DNA polymerase I, a fragment from the Bst polymerase, SEQUENASE 1.0, SEQUENASE 2.0, T5 DNA polymerase, Vent_{R}(exo-) polymerase, ThermoPhi, and bacteriophage Phi29 DNA polymerase.

15. The method according to claim 13 or 14, wherein the amplification products are branched double stranded concatamers of the vector nucleic acid species.

16. The method according to claim 15, wherein the DNA polymerase is bacteriophage phi29 DNA polymerase or a functionally equivalent polymerase, such as truncated phi29 polymerase, and a mutant of phi29 polymerase having at least 80% sequnce identity with phi29 or with a functional truncate thereof.

17. The method according to anyone of the preceding claims wherein the molecular amplification procedure is primed by random oligonucleotide primers, such as random hexamer primers.

18. The method according to any one of the preceding claims wherein the molecular amplification procedure is isothermal.

19. The method according to claim 18, wherein the temperature during the molecular amplification procedure is kept in the range between 15 and 50°C such as between 20°C and 45°C.

20. The method according to any one of the preceding claims wherein the eukaryotic cells of step c) and C) are selected from the group consisting of fungal cells, such as cells of filamentous fungi and yeast; plant cells; and animal cells.

21. The method according to any one of the preceding claims, wherein step e) and E) comprises testing for the presence of secreted EPPF in the culture medium.

22. The method according to any one of the preceding claims, wherein the EPPF is an antibody, an antibody fragment, or a synthetic or semi-synthetic antibody or antibody analogue which binds a predetermined antigen.

23. The method according to any one of the preceding claims, wherein the nucleic acids in step a) or A) are obtained from bacteria transfected with nucleic acids of which at least some are expected to encode the EPPF.

24. The method according to any of the preceding claims, wherein the plurality of vector nucleic acid species encoding an EPPF are derived through a multiplex molecular amplification procedure using separate templates derived from isolated single cells, the molecular amplification comprising generating cognate pairs of variable region encoding sequences through linking of nucleotide sequences encoding variable region each cognate pair being derived from one isolated single cell.

25. The method according to any one of the preceding claims, wherein double stranded vectors of step a) or double stranded nucleic acids of step A) are subjected to base denaturation.

26. A method for producing an EPPF in eukaryotic cells, the method comprising identifying a vector nucleic acid by means of the method according to any one of the preceding claims, and subsequently transfecting or transducing a suitable eukaryotic host cell with the vector from where the vector nucleic acid is derived, culturing the transfected or transduced eukaryotic cell under conditions which facilitate expression of the EPPF encoding nucleic acid and recovering the EPPF from the culture.

27. The method according to claim 26, wherein the EPPF is an antibody, antibody fragment or antibody analogue.
